(19) Europäisches Patentamt

European Patent Office

Office européen des brevets

(11) Publication number: **0 457 532 A1**

(12) **EUROPEAN PATENT APPLICATION**

(21) Application number: 91304280.0

(51) Int. Cl.$^5$: **C12P 21/08, A61K 39/395**

(22) Date of filing: 13.05.91

(30) Priority: 14.05.90 IT 4159990

(43) Date of publication of application:
21.11.91 Bulletin 91/47

(84) Designated Contracting States:
AT BE CH DE DK ES FR GB GR IT LI LU NL SE

(71) Applicant: FIDIA S.p.A.
Via Ponte della Fabbrica 3-A
I-35031 Abano Terme (Padova) (IT)

(72) Inventor: Della Vale, Francesco
via Cerato 14
I-35100 Padova (IT)
Inventor: Callegaro, Lanfranco
via Bravi 35
I-35020 Ponte di Brenta (IT)
Inventor: Prosdocimi, Marco
via Corso Umberto 1
Padua (IT)

(74) Representative: Pendlebury, Anthony et al
PAGE, WHITE & FARRER 54 Doughty Street
London WC1N 2LS (GB)

(54) **Monoclonal antibody which inhibits the biological action of platelet factor 4.**

(57) A new monoclonal antibody, of class IgG1, is disclosed specific for platelet factor 4 (PF4) of human and rabbit origin which has the property of selectively blocking the biological action of PF4, without interacting with or altering the other protein components of the platelet system. The present invention concerns this monoclonal antibody, its hybridoma, production and use, including diagnostic and therapeutic uses thereof.

EP 0 457 532 A1

BACKGROUND OF THE INVENTION

Thrombus formation in the veins and arteries is a pathological process leading to serious consequences for the patient. Arterial thrombosis in particular is the main cause of coronary, cerebral and peripheral ischemic pathology. The possibility of preventing arterial thrombosis by pharmacological intervention in high-risk situations has long been explored, using a variety of drugs, and important results have been obtained both in the experimental and clinical fields. Heparin and oral anticoagulants are the most widely used drugs for venous thrombosis, while for arterial thrombosis drugs able to inhibit platelet aggregation have been the most used up till now. Acetylsalicylic acid and ticlopidine are the compounds which have been most widely studied and whose actions are most clearly documented (Stein et al., J. Am. Coll. Cardiol. 14, 813-36, 1989).

In recent years the role of thrombin in the pathogenesis of arterial thrombosis has been assessed from a fresh angle and consequently the use of drugs able to inhibit this protease in the arteries has also been reconsidered. It has been observed that heparin is clinically efficacious in situations of arterial thrombotic risk (Theroux et al. , N. Engl. J. Med. 319, 1105-11, 1988; Neri Serneri et al., Lancet 335, 615-18, 1990) and the effects of specific thrombin antagonists have been investigated in experimental studies. In particular, small peptides capable of blocking the active site of thrombin and proteins of natural origin, such as hirudin, have proved active in the prevention of experimental arterial thrombosis (Heras et al., Circulation, 79, 657-65, 1989; Fitzgerald and Fitzgerald, Proc. Natl. Acad. Sci. USA 86, 7585-89, 1989; Eidt et al., J. Clin. Invest. 84, 18-27, 1989). The use of peptide-type substances is not, however, limited to the aforesaid experiments with selective thrombin blockers, it extends to another selective target of the thrombotic process. It is well known that the final stage of the platelet aggregation process is the binding of fibrinogen to one of its specific receptors, the glycoprotein IIb-IIIa (GP IIb-IIIa), which is expressed on the platelet surface after cell activation. Interaction of fibrinogen with GP IIb-IIIa can be blocked by specific monoclonal antibodies or by peptides capable of interacting with specific domains of the receptor, in particular peptides containing the RGD sequence (Ruggeri, Circulation, 80, 1920-22, 1989; Fitzgerald, Circulation, 80, 1918-19, 1989).

This type of technical advancement, both in terms of antibodies and of peptides, has made it possible to obtain, in experimental models of arterial thrombosis, important pharmacological results in the prevention of occlusion (Henkel et al., Circulation 80, 1775-82, 1989; Coller et al., Circulation 80, 1766-74, 1989; Cadroy et al., J. Clin. Invest. 84, 939-44, 1989).

SUMMARY OF THE INVENTION

The present invention, therefore, is directed to a novel monoclonal antibody which is specific for platelet factor 4 (PF4), and which blocks the biological action of PF4 without cross-reacting with any other proteins, particularly without cross-reacting with any other proteins involved in the platelet system. The antibody is useful for both diagnostic and therapeutic purposes.

For diagnostic uses, the anti-PF4 antibody can be utilized in a test kit for determining the nature or amount of in vivo generated PF4 peptide, which provides clinically useful information. One such test kit comprises an aliquot of lyophilized anti-PF4 MAb and an aliquot of PF4 to be utilized in an ELISA test to prepare a standard curve and to establish the competitive reaction mechanism utilized to determine the level of PF4 in a serum sample.

The anti-PF4 MAb of the invention can also be utilized in standard and known sandwich immunoassay procedures and RIA procedures wherein the anti-PF4 MAb is utilized as the antibody for specific binding to the PF4 peptide.

In addition, the anti-PF4 antibody, due to its specific binding to PF4, can also be utilized to remove and/or purify PF4 from serum samples. Hemodialysis can, for example, be used to effect such removal wherein the anti-PF4 MAb is immobilized on a solid support, such as glass beads. The antibody-solid support complex is then placed within the hemodialysis chamber at a location in the chamber before the blood is returned to the patient. Accordingly, during hemodialysis, the passing of the blood through the hemodialysis chamber such that the blood contacts the immobilized MAb, essentially cleanses the blood of PF4 before the blood is returned to the circulatory system of the patient. The purification can be, for example, a system such as that disclosed in U.S. Patent 4,361,509 or Re. 32,011, which are hereby incorporated by reference.

For therapeutic purposes, the anti-PF4 MAb of the invention can also be combined with a physiologically acceptable carrier or diluent and administered to a patient in an amount effective to bind and biologically inactivate sufficient amounts of PF4 so as to treat or prevent thrombosis.

An additional therapeutic use of the anti-PF4 MAb of the invention is in the field of wound healing, wherein the MAb can be combined with other known wound healing compositions in order to inhibit the anti-angiogenic action of PF4.

DETAILED DESCRIPTION OF THE INVENTION

The importance of platelets and thrombin in the thrombotic process at the arterial level suggests that modulating the interaction with the vascular wall can be highly significant in preventing vascular occlusion. During the platelet activation process, numerous modulatory substances are released from the platelet organules. In particular, a protein weighing about 8000 Daltons and known as Platelet Factor 4 (PF4) is released from the alpha-granules into the plasma. Apart from an anti-heparin action, this protein is endowed with other important biological activities such as a high affinity for the vascular endothelium, chemotaxis, immunologic modulation and regulation of the angiogenic process (Deuel et al., Proc. Natl. Acad. Sci. USA 78, 4584-4587, 1981; Prosdocimi et al., Thromb. Haemost. 52, 157-159, 1984; Niewiarowski S. and Holt J.C., in "The platelets: Physiology and Pharmacology" pages 49-83, Longenecker G.L. ed., Academic Press, Orlando 1985; Zucker et al., Proc. Natl. Acad. Sci. USA 86, 7571-7574, 1989; Maione et al., Science 247, 77-79, 1990).

On this basis, platelet aggregation and the coagulative process are therefore involved in the thrombotic phenomenon and the key factor is probably the local release of thrombin which can stimulate both further platelet aggregation and the formation of fibrin, and can favor the adhesion of white cells to the endothelium. The protease activity of thrombin is blocked by the natural antagonist Antithrombin III present in plasma. The importance of the equilibrium between thrombin and Antithrombin III activities is strengthened by the fact that the concentration of the latter in its active phase in plasma is inversely proportional to the onset of disorders of a thrombotic character.

Moreover, it is widely known that the anti-coagulant effect of heparin is mediated through its bond with Antithrombin III and subsequent induction of modulation in the structural conformation of the latter. This leads to an increase in the rate of formation of Thrombin/Antithrombin III complexes with inactivation of the protease.

Since heparin is present in circulation practically only as a result of therapeutic treatment, this effect should have no physiological impact. However, these reported facts assume particular importance in view of recently-acquired evidence demonstrating the presence of heparin-like activity on the surface of the vascular endothelium. Such substances could therefore, like heparin, speed up the formation of Thrombin/Antithrombin III complexes "in vivo" and account for the anti-coagulant properties of the vascular walls.

During the "release reaction", activated platelets secrete the protein factor known as Platelet Factor 4 or PF4, which is able to influence the Heparin-Antithrombin III-Thrombin triad. Indeed, through high-affinity sites, PF4 is able to bind to the heparin molecule, preventing the bond between Antithrombin III and Thrombin. In these conditions the protease is free to act, mostly by generating an increase in the coagulative state. Platelets can therefore modulate the anti-coagulant potential of the vessel walls, by secretion of PF4.

Interesting recent studies (Ruoslahti E. et al; Cell, 64, 867-869, 1991) confirm the importance of the role of PF4 also in relation to proteoglycans, described as modulators of growth factor activities, and other important biological activities can be attributed to platelet factor 4, including the following:

– The proven presence of high-affinity receptors for PF4 on the platelets themselves suggests intervention by the factor in the regulation of cell-cell interactions. Indeed, the activation of this receptor leads to an increase in platelet sensitivity to aggregation agents such as thrombin, ADP and arachidonic acid (Capitanio et al., Biochim. Biophys. Acta, 839, 161-73, 1985).

– Evidence showing PF4 to be chemotactic for some cells of the white series, such as neutrophils and monocytes, opens new ground for interesting speculation especially in the light of recent theories which attribute to monocytes and platelets a role of primary importance in the atherosclerotic process.

These considerations, albeit brief, are enough to justify the hypothesis of a central biological role of PF4 not only in thrombotic processes. There is therefore a need for reagents able to selectively block the biological activity of PF4, to study its biological impact thoroughly and to develop completely innovatory drugs to treat thrombotic pathologies, using these reagents to block PF4 in vivo in situations where it is active. Clearly, these reagents must selectively block PF4, without interacting with other biological components, allowing the recovery of its biological activity when the blocking reagent has been eliminated.

Attention has recently focused also on other biological activities of PF4. Indeed, a major effort is being made to develop PF4 as an antimetastatic (Sharpe R.J. et al., J. Natl. Cancer Inst. 82, 848-853, 1990) and an immunostimulating agent (Zucker M.B., et al., Proc. Natl. Acad. Sci. - USA, 86, 7571-7574, 1989). This antimetastatic activity has been explained in terms of an anti-angiogenic effect, due to a reduced rate of endothelial cell proliferation (Maione, T.E. et al., Science, 247. 77-79. 1990).

In one aspect the invention aims to obtain a specific immunological reagent, such as a monoclonal antibody (MAb), able to selectively block the biological activity of PF4, so as, for example, to prevent PF4 from blocking the action of heparin.

The invention also aims to provide an MAb which is able to block the anti-angiogenic action of PF4. As this protein is released from activated platelets, the local activation of platelets, releasing PF4, can slow down endothelial cell proliferation and angiogenesis. An agent, like the anti-PF4 MAb of the invention is, therefore, useful to block the anti-angiogenic activity of PF4, and thereby sustain and promote endothelial cell proliferation and angiogenesis. To accomplish this objective, the anti-PF4 MAb of the invention is administered in a pharmaceutical composition together with a pharmaceutically acceptable carrier or diluent, and preferably as a dermal or transdermal composition, optionally together with other known wound healing compositions and agents, such as epidermal growth factor (EGF), hyaluronic acid.

The choice of producing MAbs is essentially linked with the fact that they are specific reagents and that the technological means of obtaining them sidestep difficulties linked with the use of polyclonal antibodies, such as:

a. a lack of uniformity in antibody response between immunized animals and also between samples taken from the same animal;

b. the need for large quantities of a highly pure immunogen to eliminate cross-reactions with other proteins commonly present in PF4 preparations; and

c. greater possibility of causing immune reactions in the organism receiving the antibody preparation.

The present invention makes it possible to obtain:

1) a monoclonal antibody which specifically binds to platelet factor 4 (PF4) such as the human or rabbit variety, blocking its biological activity, such as its interaction with heparin, an antibody with the following characteristics:

a) molecular weight of about 140-160 kilo-daltons,

b) it does not cross-react with other rabbit or human platelet proteins,

c) it belongs to the IgG-type class of immunoglobulins;

2) a cloned hybridoma obtained from spleen cells of mammals immunized with rabbit PF4 and a homogeneous or heterogeneous lymphoid cell;

3) a method to produce a cloned hybridoma obtained from a spleen cell of a mammal immunized with rabbit PF4 and a homogeneous or heterogeneous lymphoid cell, allowing the aforesaid spleen cell and the aforesaid lymphoid cell to merge at cell level after their cloning;

4) a method to produce a monoclonal antibody which binds specifically to human and rabbit PF4 blocking their antiheparin biological activity, including growth of the cloned hybridoma, obtained from spleen cells of mammals immunized with the aforesaid protein and homogeneous or heterogeneous lymphoid cells, in vitro, that is, in culture medium, or in the abdomen of the mammal where the hybridoma reproduces and accumulates the monoclonal antibody;

5) a method to block specifically the biological activity, for instance of an antiheparin type, of human and rabbit PF4, by using the monoclonal antibody described in point 1, without altering the other protein components, of platelet origin or otherwise. Moreover, given the peculiar nature of monoclonal antibodies, it is possible that the same antibody may block the biological activity of the protein against which it is directed, belonging to different species, as its amino acid sequence is the same and preserved. This is the situation of PF4, for example.

Rabbit PF4, used as an immunogen, has been purified from isolated rabbit platelets according to the method described for the analogous human protein (Medici et al., Thromb. Res. 54, 277-287, 1989), modified from a previous method used for rabbit PF4 (Ginsberg et al., J. Biol. Chem. 254, 12365-12371, 1979).

Various kinds of animals - sheep, goats, guinea pigs, rats and mice, have been immunized with rabbit PF4 or protein complexes; mice especially are used to obtain monoclonal antibodies. Immunization is possible by various routes, such as subcutaneous, intraperitoneal, intravenous, intramuscular, but it is preferable for the immunogen to be injected by subcutaneous, intraperitoneal and intravenous routes, and in particular subcutaneously. Moreover, immunization intervals, doses of immunogen and administration routes are variable and depend on the immunogen used. Regarding cell fusion, methods relative to cell lines and suitable chemical reagents have long been described (Galfré G. and Milstein C., Methods Enzymology 73, 3-46, 1981). Of the analytical systems used to assess the biological activity of PF4, the most widely used is the one relative to its antiheparin activity (Lane D.A. et al., Biochem. J. 218, 725-32, 1984). One method of assessing the biological activity of PF4 is the test to determine its antiheparin activity and consequently this test is used to assess which reagents can block the biological activity of PF4. The method of neutralizing the antiheparin activity of PF4 involves preincubation of the latter with the specific monoclonal antibody in examination, to assess whether the resulting antigen-antibody bond brings about the neutralization of the antiheparin biological activity of PF4, by subsequent exposure to heparin and to the detector system.

Clearly, recognition of just one definite structural form of the analyte increases the specificity characteristics of the analytical assay and this is the first requisite for a monoclonal antibody. Consequently, the experimental significance of the blocking of biological activity, for example of the anti-heparin type, of the PF4 analyte by a monoclonal antibody, must be considered an exclusive property of the antibody itself, quite apart from its ability to recognize PF4 of one specific variety. Specificity in this case is given for the most part by the blocking of antiheparin activity by the antianalyte monoclonal antibody.

It is important to have available specific reagents which selectively block PF4 without interfering with other analogous proteins belonging to the same biological sequence.

A. Preparation of the Hybridoma and Monoclonal Antibody

1. Immunization of Mice

Four Balb/c mice aged 8 weeks, supplied by Charles River, are immunized according to the following procedure: a first immunization by intraperitoneal route (ip) with 50 ug of purified rabbit PF4, dissolved in NaCi 0.9% w/v and emulsified in complete Freund's adjuvant in a ratio of 1:2. Immunization (ip) is repeated 10 days later with another 50 ug of PF4 emulsified in incomplete Freund's adjuvant, in the same ratio. Another booster is-given ip with 50 ug of PF4 dissolved in NaCl 0.9% w/v, 15 days later.

The same dose, prepared as the third immunization, is subsequently administered monthly for three months. Immediately before fusion, 25 ug of PF4 is inoculated ip on days -3 and -2 and 25 ug by intravenous route on day -1. The mouse with the highest antibody titer is then used for fusion. This titer is assessed on serum taken from the retrobulbar area and tested by an immunoenzymatic technique discussed below under "Search for Antibody-Producing Cells".

Table 1 illustrates the immunization plan.

TABLE 1

1. INITIAL PHASE OF IMMUNIZATION

| SEQUENCE OF IMMUNIZATIONS METHOD | TIME (days) | DOSE (ug) | ADMINISTRATION | SOLUBILIZATION ROUTE |
|---|---|---|---|---|
| 1st | 0 | 50 | INTRAPERITONEAL | CFA 1:2 |
| 2nd | 10 | 50 | INTRAPERITONEAL | IFA 1:2 |
| 3rd | 25 | 50 | INTRAPERITONEAL | NaCl 0.9% |
| 4th-6th | Every 30 days | 50 | INTRAPERITONEAL | NaCl 0.9% |

2. FINAL PHASE OF IMMUNIZATION

| SEQUENCE OF IMMUNIZATIONS | TIME* (days) | DOSE (ug) | ADMINISTRATION ROUTE | METHOD OF SOLUBILIZATION |
|---|---|---|---|---|
| 1a | -3 | 25 | INTRAPERITONEAL | NaCl 0.9% |
| 2a | -2 | 25 | INTRAPERITONEAL | NaCl 0.9% |
| 3a | -1 | 25 | INTRAVENOUS | NaCl 0.9% |

Legend: CFA = Complete Freund's adjuvant
        IFA = Incomplete Freund's adjuvant
        * = The days are calculated with respect to the
        day of fusion which counts as day 0.

2) Cell Fusion

On the day of fusion, the chosen animal is sacrificed by dislocating its neck. The spleen is then removed in sterile conditions by opening the abdomen with surgical scissors and pincers, making sure not to damage any part of the intestine, since this would be a sure source of contamination. The spleen is then placed on an Eterlon mesh, which in turn is placed over a sterile beaker, and crushed with a pestle in the presence of Dulbecco's Modified Eagle's Medium (DMEM) submerged in ice. The fragments of spleen thus obtained are then further separated by delicately pipetting them with a sterile Pasteur pipette. They are then transferred to a sterile, 15-ml, polystyrene test tube. The connective residue is eliminated by simple sedimentation for 10 minutes, in an iced bath. The non-precipitated cellular suspension is transferred to another test tube and centrifuged at 1000 rpm at 4°C. The upper layer is eliminated while the cell pellet is resuspended for 10 minutes in $NH_4Cl$ at 0.83% w/v, in an iced bath to lysate the red blood cells. It is then centrifuged as before and washed again, by centrifugation and resuspension of the cell pellet in DMEM.

The non-secreting myeloma cells used for fusion belong to the NS-0 cell line. They are cultivated for a week before fusion at a low concentration ($2.5-5 \times 10^5$/ml) in DMEM containing 2mm glutamine and horse serum (HS) at 10% v/v. On the day of fusion they are gathered, centrifuged at 1000 rpm at 4°C and the pellet washed and then centrifuged three times in HS-free culture medium, in an ice bath. After the washings, the spleen and myeloma cells mixed in a ratio of 5:1 respectively, are counted in a Neubauer chamber, and made to settle together in a 50-ml polypropylene test tube, by centrifugation at 1000 rpm. The upper layer is eliminated while the pellet is gently resuspended by tapping the tube with the fingers. In a typical fusion experiment, about $80 \times 10^6$ spleen cells and $16 \times 10^6$ myeloma cells are used.

The fusion procedure is effected according to the following method: 1 ml of polyethyleneglycol (PEG) 1500 at 41% v/v, heated to 37°C is slowly added to the resuspended pellet. This is then incubated for 1 minute in a gently shaking temperature bath set at 37°C. After this, 12 ml of DMEM previously heated to 37°C are added dropwise so as to slowly dilute the PEG without causing osmotic shock. The cells, centrifuged at 1000 rpm for 10 minutes at 37°C, are resuspended in hypoxanthine medium at a concentration of $1 \times 10^{-4} M$, aminopterin at a concentration of $4 \times 10^{-7} m$ and thymidine at a concentration of $1.6 \times 10^{-5} M$. This medium is known as DMEM-HAT. The cells are sown by multiple pipette at a concentration of $1 \times 10^5$ cells/well (myeloma + spleen) in 100 ul of medium, in 96-well plates. They are placed in an incubator at 37°C in damp conditions and in the presence of 5% CO2. No cells of any kind are used to favor proliferation of the hybridomas (feeder-layer).

The hybridomas appear within 3-5 days and when the clones reach dimensions equal to about half a well (7-15 days after fusion), 50 ul of culture medium are tested for the presence of antibodies. They are screened by an immunoenzymatic test on solid phase (ELISA) (see "Search for Antibody-Producing Cells, below).

The secreting hybridomas are expanded and the culture medium used for their growth is progressively changed, first the aminopterin is eliminated using a DMEM-HT medium, subsequently a medium is used which contains 20% HS v/v, Hybridoma Growth Factor (HECS) 1:100 and 2 mM glutamine and lastly the HECS is eliminated and the percent of HS is reduced to 10%.

## 3) Search for Antibody-Producing Cells

Immunoenzymatic tests (ELISA) are effected to screen the culture medium of the hybridomas obtained by fusion, in order to assess whether such hybridomas secrete antibodies specifically directed towards human or rabbit PF4.

ELISA is effected essentially according to the method described by Engvall, Methods Enzymology, 70, 419-39, 1980: 0.1 ug of the solution of purified PF4 of human or rabbit origin, diluted in 50 ul of carbonated buffer pH 9.5, are adhered to each well in polystyrene microtitration plates for 16 hours at 4°C. When the wells have been washed three times with phosphate buffer, pH 7.2, (PBS), the wells are saturated with 200 ul of a solution formed by PBS containing bovine serum albumin (BSA), 3% w/v, and this is incubated for an hour and a half at 37°C. Incubation is then continued for 3 hours at 37°C with 50 ul of supernatant from the wells which proved positive to hybridoma growth. After three of the usual washings, in each well are placed 50 ul of the second antibody constituted by rabbit IgG directed against those of mouse, conjugated with horseradish peroxidase (anti mouse-HRP) diluted 1:1000 in a saturation buffer formed by PBS + BSA 1% w/v + Tween 20 0.05% v/v. This is incubated for 1 hour at 37°C. After three further washings with PBS, detections are made using 50 ul of chromogen substrate constituted by orthophenylene diamine and $H_2O_2$ in acetate buffer, pH 5.0, incubating for 15 minutes at room temperature. In the positive wells a yellow-orange colour develops which is blocked with 50 ul of 10% v/v sulfuric acid. The optical density reading is made at 492 nm in a photometer for immunoenzymatic assays.

## 4) Cloning and Freezing

As soon as the secreting hybridoma cells are sufficiently numerous, they undergo a cloning procedure by plating 0.5 cells per well in 96-well plates in a medium of 10% DMEM-HS, 2 mM glutamine and 1:100 HECS, so that there is cell proliferation in only 50% of the wells. The clones thus grown are retested for specificity to PF4 by immunoenzymatic tests carried out as previously described ("Search for Antibody-Producing Cells"). It is thus possible to ensure effective cloning of the secreting hybridomas. In some cases this procedure can be repeated a second time.

The secreting cell lines are permanently preserved in liquid nitrogen. To this end the cloned, secreting, hybridoma cells are gathered from the culture medium, centrifuged at 4°C at 1000 rpm for 10 minutes and resuspended in a quantity of about $5 \times 10^6$/ml in the freezing solution, formed by 90% HS and dimethylsulfoxide, 10% v/v. 1 ml of this cell suspension is then placed in 1.8-ml vials for freezing. Freezing is effected gradually by first bringing the cells to 4°C for one hour, then to -20°C for two hours, then to -80°C overnight and finally storing them in liquid nitrogen.

## B. Determining Immunoglobulin Class of Antibodies

The immunoglobulin class of the MoAbs is determined using an ELISA technique similar to the one described above as far as the first stages are concerned (insolubilization of the antigen, saturation of the wells and treatment with the first antibody). For each monoclonal antibody reacting to human or rabbit PF4, 8 replications are made, corresponding to the different isotypes of light and heavy chains to be tested. Thereafter, instead of using a second antibody of the antimouse-HRP type, a series of antibodies specific for the different light and heavy chains of the mouse immunoglobulins, produced in rabbit, are used. This passage is followed by treatment with antibodies directed against rabbit IgG, conjugated with HRP and finally the aforesaid chromogen substrate is used. For each MoAb tested, coloring appears only in 2 wells, corresponding to the light chain and heavy chain isotype, of which the selected monoclonal antibodies are formed.

## C. Production of Antibodies

To obtain significant quantities of MAbs, use is made of the ability of hybridomas, which are tumoral cells lines, to develop as tumor-ascites in the abdomen of syngeneic Balb/c mice, treated, about 20 days before cell inoculation, with 0.5 ml of tetramethylpentadecane (pristane) by ip. route. This treatment favors attachment and development of the hybridomas. The cloned, secreting, hybridoma cells are reproduced in vitro until a sufficient number of cells are obtained. They are then gathered, centrifuged in the usual way, resuspended at a concentration of $2 \times 10^6$/ml in DMEM and inoculated in quantities of about 0.5 ml ip. In 7-10 days after cell inoculation, the animals develop tumor-ascites that are removed through a needle applied to the abdomen, allowing the liquid to drip into a test tube. Then the cell suspension obtained is centrifuged to separate the corpuscular part which is eliminated. The upper layer is then stored at -20°C until needed for purification of the immunoglobulins (Igs) it contains.

## D. Purification

Purification of the Igs from the ascites is carried out by the caprilic acid technique. 10 ml of ascites are mixed with 20 ml of 60mM acetate buffer, pH 4.0 and the pH is adjusted to 4.8. Caprilic acid (Ilul/ml) is added drop by drop and then submitted to vigorous magnetic stirring at room temperature for 30 minutes. After this, it is centrifuged at 20000 rpm for 20 minutes at 10°C. Then the pellet is eliminated, while the supernatant, containing the Igs, is filtered on filter paper and dialysed for 12 hours against PBS, pH 7.4.

Finally, the protein content is determined and the degree of purity of the Igs, by polyacrylamide gel electrophoresis in the presence of sodium dodecylsulfate (SDS-PAGE).

## E. Characterization of Monoclonal Antibodies

Specificity of the monoclonal antibodies obtained is determined by the Western Blot technique. The proteins of the polyacrylamide gel are transferred to a cellulose filter according to the method of Tobwin H. et al. (Proc. Natl. Acad. Sci. USA 76, 4350-54, 1979) after sodium dodecyl sulfate polyacrylamide gel electrophoresis (SDS-PAGE) as described in Laemmli, Nature, 227, 680-85, 1970. A nitrocellulose filter is placed in contact with the polyacrylamide gel and together they are inserted between two sponges pressed between a plastic 'sandwich' which is positioned inside the transfer chamber so that the protein (human or rabbit PF4) transfers from the gel towards the nitrocellulose filter. Amperage is constant throughout the transfer at 40 mA for 1 hour and then at 100 mA for 15 hours in the presence of a cooling system which keeps the temperature at 4°C. After transfer, the nitrocellulose filter is incubated at 37°C for one hour in saturation buffer formed by NaCl 85 mm, Tris 50 mM pH 8.0 containing 3% BSA w/v to saturate the protein-free sites of the nitrocellulose. The culture containing the monoclonal antibody, suitably diluted with the same saturation buffer or with the purified Igs, is incubated with the supernatant for 2 hours at room temperature, under gentle shaking.

Three 10-minute washings are performed, each with the buffer formed by 85 mm NaCl, 50 mm Tris pH 8.0 containing 0.1% BSA w/v and 0.2% P40 Non-Idet v/v. Incubation with the second antibody (anti-mouse HRP) and subsequent washings are similar to those described for the first antibody, except for the duration of incubation, which was one hour. Diaminobenzidine is used for detection, in a quantity of 5 mg dissolved in 20 ml of 100 mm Tris 10 mM imidazole pH 7.6. To this are added 10 ul of 30% $H_2O_2$ v/v. Detection is stopped by adding distilled water for enough time to allow reaction to take place.

When tested by the aforesaid immunoenzymatic and immunoblotting techniques, 11 MAbs prove to be specific for rabbit PF4 while one monoclonal antibody recognizes human and rabbit PF4 simultaneously. Specificity of the antibodies and their isotype is shown in Table 2. In particular, a monoclonal antibody called 1OB2 appears to react in immunoenzymatic assay both with human and rabbit PF4 (Table 3).

The characteristics of the selected antibody are further investigated by the immunoblotting technique using as antigen, purified PF4 and human and rabbit platelet lysates in toto, purified human and rabbit PF4 and human and rabbit serum from platelet-free plasma. This test shows that the selected hybridoma called 1OB2 reacts strongly with purified PF4, and recognizes one single band with a molecular mass of about 8000 Daltons in the human and rabbit platelet lysate which comigrates with the purified PF4, while it is completely negative with the platelet-free human and rabbit plasma-. The monoclonal antibody in question is then inoculated in syngeneic mice to obtain Igs in ascites. These are then purified as described above, thus creating highly purified reagents which specifically block the biological activity of PF4.

## TABLE   2

| NAME | ISOTYPE | | RABBIT | | HUMAN | |
|------|---------|-------|-------|------|-------|------|
| mAb | HEAVY CHAINS | LIGHT CHAINS | ELISA | BLOT | ELISA | BLOT |
| I-HA7 | N.T. | N.T. | + | + | – | – |
| II-6H12 | GAMMA1 | k | + | + | – | – |
| II-9H2 | GAMMA1 | k | + | + | – | – |
| II-14F3 | GAMMA1 | k | + | + | – | – |
| II-5G8 | GAMMA1 | k | + | + | – | – |
| II-15H2 | GAMMA1 | k | + | + | – | – |
| II-1C12 | GAMMA2b | k | + | + | – | – |
| II-11H6 | N.T. | N.T. | + | + | – | – |
| II-12G10 | GAMMA2b | k | + | + | – | – |
| II-1OB2 | GAMMA1 | k | + | + | + | + |
| II-15F11 | GAMMA1 | k | + | + | – | – |
| II-12B3 | N.T. | N.T. | + | + | – | – |

```
Legend:    +    =   positive reaction
           -    =   negative reaction
           mAb  =   monoclonal antibody
           N.T. =   not tested
```

## TABLE 3

| ANTIBODY | ABSORBANCE AT 492 nm | | | |
|---|---|---|---|---|
| CONCENTRATION | 1OB2 | | 15F11 | |
| [ug/ml] | hPF4 | raPF4 | hPF4 | raPF4 |
| 150 | 0.352 | 0.452 | 0.032 | 0.497 |
| 75 | 0.343 | 0.430 | 0.008 | 0.455 |
| 37.5 | 0.191 | 0.367 | 0.012 | 0.415 |
| 18.75 | 0.118 | 0.345 | 0.014 | 0.349 |
| 9.37 | 0.093 | 0.306 | 0.016 | 0.265 |
| 4.70 | 0.046 | 0.210 | 0.016 | 0.232 |
| 2.35 | 0.010 | 0.026 | 0.003 | 0.126 |
| 1.18 | 0.027 | 0.000 | 0.000 | 0.067 |
| 0.59 | 0.000 | 0.000 | 0.000 | 0.000 |

Legend: hPF4 = human Platelet Factor 4
raPF4 = rabbit Platelet Factor 4

### F. Measuring Biological Activity

The method used to determine the neutralization of heparin activity by PF4 is essentially that described by Lane et al., Biochem. J., 218, 725-32, 1984. 1.5-ml Eppendorf test tubes are saturated with 500 ul of PBS containing 3% BSA for 16 hours at 4°C. The excess PBS-BSA is then eliminated and in these test tubes is preincubated a fixed quantity of rabbit or human PF4 (0.2 ug) (which is able to inhibit the anti Xa Factor activity of 0.04 ug of standard heparin by 90%) with gradually decreasing doses of monoclonal antibody starting from 33 ug.

Incubation is conducted at 37°C for 2 hours in a volume of 20 ul of 0.15 M NaCl buffer, 50 mM Tris pH 8.4. At the end of the incubation period 0.04 ug of heparin are added, reaching a final volume of 180 ul. This is incubated for another two hours at 37 °C. To this mixture are added 20 ul of human Antithrombin III (Kabi Vitrum) at a concentration of 250 ug/ml, it is mixed and divided into aliquots of 50 ul in microtitration plates. It is incubated for 5 minutes at 37°C. 25 ul of bovine Xa Factor (Kabi Vitrum) with an activity of 7 nkat S-2222/ml are added to each well and incubated at 37 °C for one minute. 50 ul of S-2222 substrate are added at a concentration of 1 mM/1 to each well and incubated for 4 minutes at 37°C. The reaction is blocked with 75 ul of 20% acetic acid v/v.

The coloring which develops is read at 405 nm. Neutralization of the antiheparin activity of PF4 is expressed in percent considering 0% as the anti-heparin activity of a system constituted by MAb 1OB2, heparin and AT III (that is, in the absence of PF4) and 100% as the anti-heparin activity of the system formed by heparin, AT III and PF4 (that is, in the absence of MAb). Table 4 reports the data on neutralization of the anti-heparin activity of human and rabbit PF4 obtained with different doses of MAb 1OB2 in comparison to another MAb, called 15F11, which recognizes rabbit PF4 but does not block its biological activity. The MAb 1OB2 is able to neutralize the anti-heparin activity of human and rabbit PF4 at doses as low as 3.6 ug, that is, at molar ratios of about 1:1 with regard to PF4.

TABLE 4

PERCENT OF NEUTRALIZATION

| mAb | OF ANTI-HEPARIN ACTIVITY* | | |
|---|---|---|---|
| ADDED | | 10B2 | 15F11 |
| [ug] | RABBIT PF4 | HUMAN PF4 | RABBIT PF4 |
| 33.00 | 100.8± 1.5 | 97.0± 1.0 | -4.0±2.1 |
| 11.00 | 95.8± 0.8 | 98.0± 3.0 | 4 ±3.2 |
| 3.60 | 95.3± 0.9 | 94.5± 1.5 | 2.5±3.5 |
| 1.20 | 32.5±15.1 | 62.5±10.5 | -1.5±3.0 |
| 0.40 | -1.3± 4.0 | 4.5± 4.5 | 3 ±4.4 |
| 0.13 | 0.8± 3.3 | -3.0± 6.0 | 4 ±1.5 |

Values expressed as means ± standard error of the mean.

Information Regarding Deposit of Biological Material

The hybridoma identified above as 10B2 has been deposited in the European Collection of Animal Cell Cultures (ECACC) under the provisions of the Budapest Treaty as provisional accession number 91050801.

The invention being thus described, it is clear that the described methods can be modified in various ways. Such modifications are not to be considered as divergences from the spirit and purpose of the invention and any modification which would appear evident to the expert in the field comes within the scope of the following claims.

**Claims**

1. A monoclonal antibody which
   a) is of the IgG class;
   b) reacts with human PF4;
   c) blocks the biological action of human PF4 as compared to heparin; and
   d) does not react with any protein component other than PF4 present in platelet lysates or with any protein in platelet-free plasma.

2. The monoclonal antibody according to claim 1, which also reacts with rabbit PF4, but does not react with any protein content other than PF4 present in rabbit platelet lysate or with any protein in platelet-free rabbit plasma.

3. A hybridoma which produces the monoclonal antibody according to claim 1.

4. The hybridoma according to claim 3, which is the clone 10B2.

5. A monoclonal antibody produced by a hybridoma formed by fusion of cells from the mouse myeloma NS-O line, and spleen cells from mice previously immunized with PF4.

6. A monoclonal antibody obtainable by the process which comprises the following steps:
   a) immunizing of mice with PF4;
   b) removing the spleens of said mice, and effecting suspension of the cells thereof;
   c) fusing said spleen cells with mouse myeloma cells in the presence of a fusion promoter;
   d) diluting and culturing of said fused cells in separate wells in a culture medium which does not allow the growth of unfused cells;
   e) assessing the supernatant of each well containing the hybridoma for the presence of antibodies for PF4;
   f) selecting and cloning of the antibody-producing hybridoma; and
   g) obtaining the desired antibody from the supernatant of said selected clone.

7. The monoclonal antibody according to claim 6, wherein the process further comprises:
   a) transferring said hybridoma clone into mouse peritoneum; and
   b) recovering the ascites from said mice, said ascites containing the desired antibody.

8. The monoclonal antibody according to claim 7, produced by the process which further comprises introducing the hybridoma into a fermentation system and recovering the antibody from the culture medium used.

9. The monoclonal antibody according to claim 7 or 8, wherein the hybridoma is the clone 10B2.

10. The monoclonal antibody according to any one of claims 7 to 9, which reacts with human PF4.

11. The monoclonal antibody according to claim 10, which blocks the biological activity of human PF4.

12. The monoclonal antibody according to claim 11, where the biological activity of PF4 is of the anti-heparin type.

13. The monoclonal antibody according to claim 10, wherein the antibody does not cross-react with any platelet component of human origin.

14. The monoclonal antibody according to claim 10, wherein the antibody does not cross-react with any component of platelet-free plasma of human origin.

15. A method of producing a monoclonal antibody which is of the IgG class, reacts with human PF4, blocks the biological action of human PF4 and does not cross react with any platelet component or with any component of platelet-free plasma of human origin, which process comprises:
   a) immunizing of mice with PF4;
   b) removing the spleens of said mice, and effecting suspension of the cells thereof;
   c) fusing said spleen cells with mouse myeloma cells in the presence of a fusion promoter;
   d) diluting and culturing of said fused cells in separate wells in a culture medium which does not allow the growth of unfused cells;
   e) assessing the supernatant of each well containing the hybridoma for the presence of antibodies for PF4;
   f) selecting and cloning of the antibody-producing hybridoma; and
   g) obtaining the desired antibody from the supernatant of said selected clone.

16. The method according to claim 15, which further comprises transferring said hybridoma into mouse peritoneum and recovering the ascites from said mice, said ascites containing the desired antibody.

17. A pharmaceutical composition which comprises a pharmaceutically acceptable carrier or diluent and the monoclonal antibody according to any one of claims 1-2 or 6-8 in an amount sufficient to slow the thrombotic process.

18. A composition comprising an effective amount of a monoclonal antibody according to any one of claims 1-2 or 6-8 for use in a method for preventing or treating thrombosis.

19. A composition comprising an anti-PF4 monoclonal antibody in an amount sufficient to block the anti-angiogenic activity of PF4 for use in a method for enhancing wound healing.

20. A composition according to claim 19, wherein the antibody is the monoclonal antibody according to any one of claims 1-2 or 6-8.

21. Use of an anti-PF4 monoclonal antibody according to claim 1 to slow down formation of the thrombotic process.

22. Use of an anti-PF4 monoclonal antibody according to claim 1, in association with compounds having antithrombotic or thrombolytic action.

23. Use of the anti-PF4 monoclonal antibody according to claim 1 as a pro-angiogenic agent.

24. Use of the anti-PF4 monoclonal antibody according to any one of claims 1-2 or 6-8 as a pro-angiogenic agent.

25. Use according to claim 23 or 24 wherein the monoclonal antibody is the antibody 10B2.

26. Use according to any one of claims 23-25, wherein said antibody is used in association with at least one compound having anti-angiogenic action.

European Patent Office

**PARTIAL EUROPEAN SEARCH REPORT**
which under Rule 45 of the European Patent Convention shall be considered, for the purposes of subsequent proceedings, as the European search report

Application number

**EP 91 30 4280**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-85 04 397 (ONCOGEN)<br><br>* Page 16, line 10 - page 17, line 35, page 30, line 16 - page 32, line 5 * | 1-20 | C 12 P 21/08<br>A 61 K 39/395 |

----

TECHNICAL FIELDS
SEARCHED (Int. Cl.4)

C 07 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:    1-20

Claims searched incompletely:

Claims not searched:    21-26

Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 02-08-1991 | REMPP |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1. 03.82